# EUROPÄISCHE PATENTSCHRIFT

(11) **EP 2 875 844 B1**
(45) Veröffentlichungstag und Bekanntmachung des Hinweises auf die Patenterteilung: **03.01.2018**
(21) Anmeldenummer: 14189633.2
(22) Anmeldetag: 21.10.2014
(51) Int. Cl.: A61N 1/05, A61N 1/08, A61N 1/37

(54) **Implantierbare Elektrodeneinrichtung, insbesondere für kardiologische Geräte, wie Herzschrittmacher**
Implantable electrode arrangement, in particular for cardiological devices, such as cardiac pacemakers
Dispositif d'électrodes implantables, notamment pour appareils cardiologiques, comme un stimulateur cardiaque

(30) Priorität: 25.11.2013 US 201361908183 P
(43) Veröffentlichungstag der Anmeldung: 27.05.2015
(73) Patentinhaber: BIOTRONIK SE & Co. KG, 12359 Berlin (DE)
(72) Erfinder: Rump, Jens, 12049 Berlin (DE); Friedrich, Michael, 14532 Kleinmachnow (DE); Büssing, Heinrich, 10317 Berlin (DE)
(74) Vertreter: Galander, Marcus

(56) Entgegenhaltungen:
- EP-A1- 2 602 001
- EP-A2- 2 478 932
- DE-A1-102011 086 208
- DE-C1- 19 840 211
- US-A1- 2004 215 279
- US-A1- 2011 137 390

## Beschreibung

Die Erfindung betrifft eine implantierbare Elektrodeneinrichtung, insbesondere für kardiologische Geräte, wie Herzschrittmacher, die einen langgestreckten Elektrodenkörper mit einem proximalen und einem distalen Ende, mindestens eine Elektrode mit einer mit dem Körper des Implantatträgers in Kontakt bringbaren Elektrodenfläche im Bereich des distalen Endes des Elektrodenkörpers und mindestens eine elektrische Kontaktleitung zur Kontaktierung der Elektrode umfasst.

Zum Hintergrund der Erfindung ist festzuhalten, dass bei solchen implantierbaren Elektrodeneinrichtungen die Kontaktleitung(en) bei Einstrahlung elektromagnetischer Wellen ähnlich einer Antenne wirken, die die aufgenommene Energie in Wärme umwandeln. Ein typisches Beispiel für diese Problematik ist die Untersuchung von Patienten mit Herzschrittmachern in Magnetresonanz-Tomographen, die neben einem statischen Magnetfeld ein damit überlagertes, hochfrequentes Wechsel-Magnetfeld einsetzen. Wird die implantierbare Elektrodeneinrichtung einem solchen Wechsel-Magnetfeld ausgesetzt, treten vorzugsweise an den Enden der im Implantat verlaufenden Kontaktleitungen Erhitzungserscheinungen auf, die von der Amplitude der eingestrahlten elektromagnetischen Welle abhängig sind und bei Ausbildung stehender Wellen maximal werden.

Zur Lösung dieser Problematik sind aus dem Stand der Technik verschiedene Ansätze bekannt. So offenbart die US 7 363 090 B2 Filtereinrichtungen in den zu einer Tip- bzw. Ringelektrode führenden Zuleitung in Form von elektromagnetischen Schwingkreisen, die mit Hilfe üblicher R-, C- und L-Glieder aufgebaut sind. Durch die Integration in die Kontaktleitung haben derartige Filterelemente zwangsläufig Einfluss auf die Elektrodenfunktionalität.

Ein weiterer Grundansatz besteht in der Verwendung sogenannter Shunts, wie dies die US 2008/00009905 A1, US 6 944 489 B2 oder US 6 871 091 B2 offenbaren. Der Shunt bildet eine für Gleichspannungssignale isolierende Ableitung von der Kontaktleitung, die aufgrund ihrer Bauart allerdings hochfrequente Signale leiten kann, wie dies typischerweise durch Kondensatorelemente realisiert ist. Damit wird die Kontaktleitung kapazitiv an das um die Elektrodeneinrichtung liegende Gewebe angekoppelt, so dass bei Einstrahlung eines hochfrequenten Signals die von der Kontaktleitung aufgenommene Energie über den Shunt über eine größere Fläche verteilt an das umliegende Gewebe abgegeben wird. Da die Energie der eingestrahlten Signale in ihrem Wesen nicht umgesetzt wird, sondern im Hinblick auf die kapazitive Kopplung wiederum ein elektrisches Wechselfeld erzeugt wird, ist die erreichbare Dissipationsrate für die eingestrahlte Energie begrenzt.

Die Europäische Patentanmeldung EP 2 478 932 A2 beschreibt ein temporäres oder permanent implantierbares medizinisches Gerät mit wenigstens einem langgestreckten elektrischen Funktionsleiter für die Übertragung von Therapiesignalen oder Diagnosesignalen oder beidem.

Weiterhin beschreibt die EP 2 602 001 A1 ein medizintechnisches Implantat mit einem bei elektrischer und/oder magnetischer Ansteuerung das Implantat in mechanische Schwingungen versetzenden Schwingerelement.

Davon ausgehend liegt der Erfindung die Aufgabe zugrunde, eine implantierbare Elektrodeneinrichtung so zu verbessern, dass in die Elektrodenleitung(en) eingestrahlte elektromagnetische höherfrequente Signale effektiver umgesetzt und körperverträglich verteilt werden können.

Diese Aufgabe wird durch die im Kennzeichnungsteil des Anspruches 1 angegebenen Merkmale gelöst, wonach eine an der Elektrode oder Kontaktleitung angeschlossene elektromechanische Resonanzeinrichtung zur Umwandlung in die Elektrodeneinrichtung eingestrahlter hochfrequenter Signale in akustomechanische Schwingungen vorgesehen ist.

Mit Hilfe der erfindungsgemäßen Lösung ist es möglich, die eingestrahlte elektromagnetische Energie in eine völlig andere Energieform, nämlich mechanische Energie in Form von Schallwellen, umzusetzen, wodurch eine sehr körperverträgliche, gewebeschonende Energiedissipation stattfinden kann.

Vorzugsweise weist die elektromagnetische Resonanzeinrichtung ein piezokristallines Resonanzelement auf. Diese stellen typische Vertreter elektromechanischer Resonanzkörper dar, die in der Regel aus Keramikmaterialien mit sogenannter nichtzentrosymmetrischer Punktgruppe bestehen. Vorzugsweise haben solche piezokristallinen Resonanzelemente dann eine geometrische Abmessung, die der halben Wellenlänge der akustomechanischen Schwingungen in einem Frequenzbereich von 10 MHz bis 400 MHz entspricht. Dieser Frequenzbereich ist die typische Bandbreite für Frequenzen, die es gilt, bei solchen implantierbaren Elektrodeneinrichtungen zu berücksichtigen, um die eingangs erwähnten Probleme mit Hochfrequenzsignalen zu beheben. Auch ungeradzahlige Vielfache der halben Wellenlänge der akustomechanischen Schwingung können als Basis für die geometrische Abmessung des piezokristallinen Resonanzelementes dienen. Eine entsprechende Komponente kann also je nach Schwingungstyp beispielsweise einen Durchmesser haben, der der halben Wellenlänge von Schallwellen in dem oben erwähnten Radiofrequenzbereich entspricht.

In einer grundlegenden Konfiguration kann das piezokristalline Resonanzelement ein hülsenförmiges Piezokristallelement sein. Dies lässt sich besonders gut mit den üblichen Elektrodenformen, wie einer Tip-Elektrode mit einer seitlichen Ringfläche oder einer Ringelektrode selbst kombinieren.

Für die Auslegung der Ringdicke eines solchen hülsenförmigen Piezokristallelements kann wiederum die halbe Wellenlänge der akustomechanischen Schwingung als Dimensionsvorgabe dienen. Praktische Werte liegen dann beispielsweise für eine 64 MHz-Schallwelle mit einer Wellenlänge von 200 µm der Druckwelle bei einer Resonanzschichtdicke von etwa 100 µm. Derartige Wandstärken sind mit der gewünschten Kristallstruktur grundsätzlich herstellbar.

Eine alternative Auslegung für die elektromechanische Resonanzeinrichtung liegt in der Ausbildung als piezokristalline Folie, insbesondere Folien, die Teilweise oder ganz aus PVDF (Polyvinylidenfluorid) bestehen, im weiteren PVDF Folien oder Polyvinylidenfluoridfolien. PVDF ist ein opaker, teilkristalliner, thermoplastischer Fluorkunststoff mit piezo-elektrischen Eigenschaften.

Eine weitere alternative Auslegung liegt in der Ausbildung einer piezokristalline Folie durch das Auftragen von Materialien mit piezokristalline Eigenschaften auf eine Folie mittels PVD (Physical Vapour Deposition) Verfahren.

Vorzugsweise weisen piezokristalline Resonanzelemente in Folienform eine Schichtbauweise zusammengesetzt aus der piezokristallinen Folie und einer Metallschicht auf. Diese Metallschicht dient als Anschlussfläche des Resonanzelementes. In bevorzugter Weiterbildung kann dieser Schichtaufbau in eine echte Sandwichbauweise ausgebaut werden, wobei dann mindestens zwei sich abwechselnde Folien- und Metallschichten vorgesehen sind.

Ein derartiges Resonanzelement kann in herstellungstechnisch einfacher Weise bei einer erfindungsgemäßen Elektrodeneinrichtung realisiert werden, indem die piezokristalline Folie mit der Metallschicht zur Bildung der elektromechanischen Resonanzeinrichtung mehrfach um einen Träger, insbesondere um eine Kopfhülse einer Tipelektrode, gewickelt ist. Die äußere Metallisierung dieses piezokeramischen Elementes stellt dann den elektrischen Kontakt zum Körper, also insbesondere Körpergewebe oder -flüssigkeiten, her.

Eine solche Folien-Metall-Schichtkonfiguration - auch bei Ausbildung als Mehrfachwicklung - ist besonders gut mit Hilfe einer Anschlussfahne kontaktierbar.

Die schaltungstechnische Anordnung der elektromechanischen Resonanzeinrichtung, liegt zwischen einer Elektrode oder ihrer Kontaktleitung und dem Körper des Implantatträgers selbst. Im Bereich der distalen Elektrode kann dabei die elektromechanische Resonanzeinrichtung außen auf der Kopfhülse oder innen an der Kopfhülse oder auch innerhalb der Kopfhülse auf einer Welle zur Betätigung einer Verankerungsschraube an der Tip-Elektrode angeordnet sein.

Auch eine Positionierung unter der Ringelektrode ist denkbar.

Schließlich ist das piezokristalline Resonanzelement ganz oder teilweise aus einem oder mehreren der folgenden Materialien hergestellt: anisotropes SiO₂ (α- Quarz Glas), Aluminium-Nitrid, Polyvinylidenfluorid, Barium-Titanat, Lithiumniobate, Galliumorthophosphate, Berlinit, Mineralien der Turmalingruppe, Seignettesal oder Kalium-Natrium-Niobate.

Zusammenfassend werden durch die gewählten elektromechanischen Resonanzelemente und deren Verbauung in implantierbaren Elektrodeneinrichtungen die funktionellen Eigenschaften insbesondere im regulären Gleichsignalbetrieb der Elektroden durch die erfindungsgemäßen Maßnahmen nicht gestört. Gleichzeitig kann jedoch eine wirkungsvolle Reduzierung der Erwärmung am Ende der Kontaktleitungen aufgrund eingestrahlter elektromagnetischer Wechselfelder erreicht werden.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: eine schematische Darstellung einer implantierbaren Elektrodeneinrichtung nach Art eines Ersatzschaltbildes für die elektrischen Komponenten,
- Fig. 2 und 3: schematische, teilweise geschnittene Ansichten des distalen Endes einer Elektrodeneinrichtung in unterschiedlichen Ausführungsformen der elektromechanischen Resonanzeinrichtung auf der Basis eines hülsenförmigen Piezokristallelements,
- Fig. 4: eine Ansicht einer Elektrodeneinrichtung analog Fig. 2 und 3 mit einem piezokristallinen Resonanzelement auf der Basis einer piezokristallinen Folie, sowie
- Fig. 5 und 6: eine Draufsicht und einen Schnitt durch eine Sandwich-Konstruktion einer piezokristallinen Folie mit Metallschicht zum Einsatz in der Elektrodeneinrichtung gemäß Fig. 4.

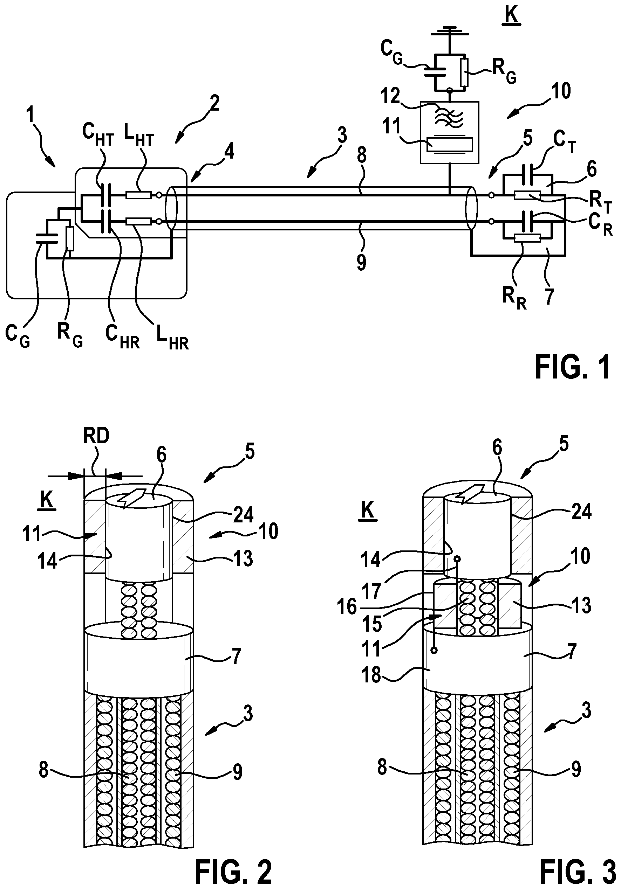

Fig. 1 zeigt eine implantierbare Elektrodeneinrichtung mit einem schematisch dargestellten Herzschrittmacher 1, an dessen Header 2 ein langgestreckter Elektrodenkörper 3 mit seinem proximalen Ende 4 angeschlossen ist. Am distalen Ende 5 des Elektrodenkörpers 3 befindet sich eine Tip-Elektrode 6 direkt an der Spitze des Elektrodenkörpers 1 sowie in proximaler Richtung dahinter eine Ringelektrode 7. Derartige Elektrodenanordnungen sind üblich und bedürfen keiner näheren Erläuterung. Im Elektrodenkörper 3 verlaufen die in der Regel wendelförmigen Kontaktleitungen 8 bzw. 9 für die Tip-Elektrode 6 bzw. die Ringelektrode 7.

Die nach Art eines Ersatzschaltbildes ausgeführte Fig. 1 gibt im Bereich des Headers 2 die kapazitiven und Impedanz-Widerstände C_{HT}, L_{HT}, C_{HR}, L_{HR} zu den Kontaktleitungen 8, 9 sowie die kapazitiven und Ohmschen Widerstände C_{G}, R_{G} zum Elektrodenkörper selbst dar.

Am distalen Ende 5 des Elektrodenkörpers 3 sind die kapazitiven Widerstände C_{T} bzw. C_{R} sowie die Ohmschen Übergangswiderstände R_{T} bzw. R_{R} der Tip- und Ringelektrode 6, 7 dargestellt.

An die Kontaktleitung 8 der Tip-Elektrode 6 ist nun eine elektromechanische Resonanzeinrichtung 10 angeschlossen, die ein piezokristallines Resonanzelement 11 aufweist. Etwaige in die Kontaktleitung 8 eingestrahlten hochfrequenten Signale regen das Resonanzelement 11 zu mechanischen Schwingungen, also Schallwellen 12, an, die an das Gewebe abgegeben werden. Der Kontakt zwischen elektromechanischer Resonanzeinrichtung 10 und dem Gewebe des Körpers, in dem die Vorrichtung implantiert ist, ist durch die Kapazität C_{G} bzw. den Ohmschen Widerstand R_{G} in Ersatzschaltbild gemäß Fig. 1 versinnbildlicht.

Bei der in Fig. 2 gezeigten Ausführungsform weist der Elektrodenkörper 3 die bereits erwähnte Tip- und Ringelektrode 6, 7 am distalen Ende 5 auf. Die elektromechanische Resonanzeinrichtung 10 ist in Form eines hülsenförmigen Piezokristallelements 13 auf die Mantelfläche der Kopfhülse 14 der Tip-Elektrode 6 gesetzt. Die Ringdicke RD des Piezokristallelements 13 beträgt dabei die halbe Wellenlänge der resonanten Druckwellen. Die hohen Schallgeschwindigkeiten innerhalb der kristallinen Keramiken des Piezokristallelements 13 von beispielsweise 10 km/s ergeben bei einer Frequenz von 64 MHz Druck-Wellenlängen um 200 µm. Die resonante Schichtdicke und entsprechend die Ringdicke RD des Piezokristallelements 13 liegt dann bei mindestens 100 µm. Die erzeugten Schallwellen 12 werden in den Körper K auf verträgliche Weise abgegebenen.

Bei der in Fig. 3 gezeigten Anordnung ist das hülsenförmige Piezokristallelement 13 zwischen Tip-Elektrode 6 und Ringelektrode 7 um die wendelförmige Kontaktleitung 8 angeordnet. Über entsprechende Kontaktflächen 15, 16 auf der inneren bzw. äußeren Mantelfläche des hülsenförmigen Piezokristallelements 13 und über Anschlussverbinder 17, 18 erfolgt der elektrische Kontakt mit der Kopfhülse 14 der Tip- bzw. mit der Ringelektrode 6 bzw. 7. Durch diese Konfiguration können jedwede in die Kontaktleitungen 8, 9 eingestrahlten hochfrequenten elektromagnetischen Signale in akustomechanische Wellen umgewandelt und die eingestrahlte Energie körperverträglich dissipiert werden.

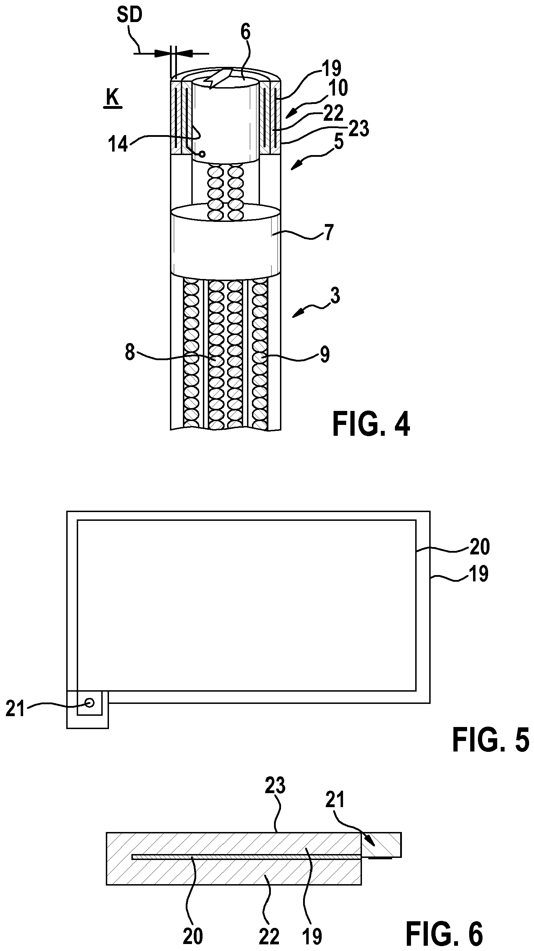

Die Fig. 4 bis 6 stellen eine weitere Ausführungsform des piezokristallinen Resonanzelementes 11 dar, das auf der Basis einer Sandwich-Konstruktion einer metallisierten piezokristallinen Folie realisiert wird. Dazu wird eine piezokristalline Folie 19 auf einer Seite mit einer Metallisierungsbeschichtung 20 versehen, die an einer Stelle mit einer Anschlussfahne 21 versehen ist. Eine zweite Schicht einer piezokristallinen Folie 23 wird auf diese Metallisierungsbeschichtung 20 aufgesetzt. Auf eine der beiden freiliegenden Oberflächen der Folien 19, 22, beispielsweise auf die in Fig. 6 obenliegende Fläche der Folie 19, wird eine weitere Metallisierungsbeschichtung 23 aufgebracht. Eine solche Sandwich-Konfiguration wird dann zuerst im Bereich der Anschlussfahne 21 mit der die Tip-Elektrode 6 bildenden Kopfhülse 14 im Bereich deren Mantelfläche verbunden und anschließend die Sandwich-Konstruktion eng um den Kopf gewickelt, so dass die Metallisierungsbeschichtung 23 außen zu liegen kommt und damit den elektrischen Kontakt zum umliegenden Gewebe oder dem Blut im Körper K des Implantatträgers herstellt. Durch die Anzahl der Wicklungen lässt sich der Resonanzeffekt beeinflussen und die Bandbreite der damit erfassbaren Frequenzen eingestrahlter elektromagnetischer Signale den Anforderungen entsprechend anpassen. Die piezokristallinen Folien 19, 22 werden durch physikalische Dampfabscheidung (PVD) hergestellt.

Die Schichtdicke SD der piezokristallinen Folie 19, 22 entspricht wiederum der halben Wellenlänge der durch die Umwandlung eingestrahlter hochfrequenter Signale erzeugten akustomechanischen Schwingungen.

## Patentansprüche

1. Implantierbare Elektrodeneinrichtung, insbesondere für kardiologische Geräte, wie Herzschrittmacher, umfassend
- einen lang gestreckten Elektrodenkörper (3) mit einem proximalen (4) und distalen Ende (5),
- mindestens einer mit dem Körper des Implantatträgers in Kontakt bringbaren Elektrode (6, 7) am oder in der Nähe des distalen Endes (5) des Elektrodenkörpers (3), und
- mindestens eine elektrische Kontaktleitung (8, 9) zur Kontaktierung der Elektrode (6, 7), - eine an Elektrode (6, 7) oder Kontaktleitung (8, 9)
angeschlossene elektromechanische Resonanzeinrichtung (10) zur Umwandlung von in die Elektrodeneinrichtung eingestrahlten hochfrequenten Signale in
akustomechanische Schwingungen (12)
**dadurch gekennzeichnet, dass**
- die elektromechanische Resonanzeinrichtung (10) schaltungstechnisch zwischen der Elektrode (6) oder ihrer Kontaktleitung (8) und dem Körper (K) des Implantatträgers angeordnet ist.

2. Elektrodeneinrichtung nach Anspruch 1, **dadurch gekennzeichnet, dass** die elektromechanische Resonanzeinrichtung (10) ein piezokristallines Resonanzelement (11) aufweist.

3. Elektrodeneinrichtung nach Anspruch 2, **dadurch gekennzeichnet, dass** das piezokristalline Resonanzelement (11) eine geometrische Abmessung (RD, SD) aufweist, die der halben Wellenlänge oder einem ungeradzahligen Vielfachen der halben Wellenlänge der akustomechanischen Schwingungen in einem Frequenzbereich von 10 MHz bis 400 MHz entspricht.

4. Elektrodeneinrichtung mindestens nach Anspruch 2, **dadurch gekennzeichnet, dass** das piezokristalline Resonanzelement (11) ein hülsenförmiges Piezokristallelement (13) ist.

5. Elektrodeneinrichtung nach Anspruch 4, **dadurch gekennzeichnet, dass** die Ringdicke (RD) des hülsenförmigen Piezokristallelements (13) der halben Wellenlänge der akustomechanischen Schwingung entspricht.

6. Elektrodeneinrichtung mindestens nach Anspruch 2, **dadurch gekennzeichnet, dass** das piezokristalline Resonanzelement (11) durch eine piezokristalline Folie (19, 22) aus PVDF, Polyvinylidenfluorid, gebildet ist.

7. Elektrodeneinrichtung nach Anspruch 6, **dadurch gekennzeichnet, dass** die piezokristalline Folie (19, 22) mit einer Metallschicht (20, 23) belegt ist.

8. Elektrodeneinrichtung nach Anspruch 7, **dadurch gekennzeichnet, dass** die piezokristalline Folie (19, 22) mit der Metallschicht (20, 23) als Sandwich-Konstruktion mit mindestens zwei sich abwechselnden Folien- und Metallschichten (19, 20, 22, 23) ausgeführt ist.

9. Elektrodeneinrichtung nach Anspruch 7 oder 8, **dadurch gekennzeichnet, dass** die piezokristalline Folie (19, 22) mit der Metallschicht (20, 23) zur Bildung der elektromechanischen Resonanzeinrichtung (10) mehrfach um einen Träger, insbesondere um eine Kopfhülse (14) einer Tipelektrode (6), gewickelt ist.

10. Elektrodeneinrichtung nach Anspruch 7 oder 8, **dadurch gekennzeichnet, dass** die Metallschicht (20) mit einer Anschlussfahne (21) zur Kontaktierung versehen ist.

11. Elektrodeneinrichtung nach einem der vorgenannten Ansprüche mit einer Kopfhülse (24) im Bereich der distalen Elektrode (6, 7), **dadurch gekennzeichnet, dass** die elektromechanische Resonanzeinrichtung (10) außen auf der Kopfhülse (14) angebracht ist oder einen Abschnitt der Kopfhülse (14) bildet.

12. Elektrodeneinrichtung nach einem der vorgenannten Ansprüche, **dadurch gekennzeichnet, dass** das piezokristalline Resonanzelement (11) ganz oder teilweise aus einem Material ausgewählt aus der folgenden Gruppe besteht: anisotropen SiO₂ (α- Quarz Glas), Aluminium-Nitrid, Polyvinylidenfluorid, Barium-Titanat, Lithiumniobate, Galliumorthophosphate, Berlinit, Mineralien der Turmalingruppe, Seignettesalz oder Kalium-Natrium-Niobate.

## Claims

1. An implantable electrode device, in particular for cardiological devices, such as cardiac pacemakers, comprising:
- an elongate electrode body (3) with a proximal end (4) and a distal end (5);
- at least one electrode (6, 7) that can be placed in contact with the body of the implant carrier and that is arranged at, or in the vicinity of, the distal end (5) of the electrode body (3); and,
- at least one electric contact line (8, 9) for contacting the electrode (6, 7),
- an electromechanical resonance device (10) connected to electrode (6, 7) or contact line (8, 9) for converting high-frequency signals irradiated into the electrode device to acousto-mechanical vibrations (12),
**characterized in that**
- the electromechanical resonance device (10) is arranged circuitry-wise between the electrode (6) or its contact line (8) and the body (K) of the implant carrier.

2. The electrode device according to claim 1, **characterized in that** the electromechanical resonance device (10) comprises a piezocrystalline resonance element (11).

3. The electrode device according to claim 2, **characterized in that** the piezocrystalline resonance element (11) comprises a geometrical dimension (RD, SD) that corresponds to a half-wavelength or an odd-numbered multiple of the half-wavelength of the acousto-mechanical vibrations in a frequency range from 10 MHz to 400 MHz.

4. The electrode device according to at least claim 2, **characterized in that** the piezocrystalline resonance element (11) is a sleeve-shaped piezocrystal element (13).

5. The electrode device according to claim 4, **characterized in that** the ring thickness (RD) of the sleeve-shaped piezocrystal element (13) corresponds to the half-wavelength of the acousto-mechanical vibration.

6. The electrode device according to at least claim 2, **characterized in that** the piezocrystalline resonance element (11) is formed by a piezocrystalline film (19, 22) made of PVDF, polyvinylidene fluoride.

7. The electrode device according to claim 6, **characterized in that** the piezocrystalline film (19, 22) is covered with a metal layer (20, 23).

8. The electrode device according to claim 7, **characterized in that** the piezocrystalline film (19, 22) having the metal layer (20, 23) is embodied as a sandwich construction having at least two alternating film and metal layers (19, 20, 22 23).

9. The electrode device according to claim 7 or 8, **characterized in that** the piezocrystalline film (19, 22) having the metal layer (20, 23) is wound a number of times around a support, especially around a head sleeve (14) of a tip electrode (6), to form the electromechanical resonance device (10).

10. The electrode device according to claim 7 or 8, **characterized in that** the metal layer (20) is provided with a connecting lug (21) for contacting.

11. The electrode device according to any of the preceding claims having a head sleeve (24) in the region of the distal electrode (6, 7), **characterized in that** the electromechanical resonance arrangement (10) is attached externally on the head sleeve (14) or forms a portion of the head sleeve (14).

12. The implantable electrode device according to any of the preceding claims, **characterized in that** the piezocrystalline resonance element (11) completely or partially comprises a material selected from the following group: anisotropic SiO₂ (α-quartz glass), aluminum nitride, polyvinylidene fluoride, barium titanate, lithium niobates, gallium orthophosphates, berlinite, minerals of the tourmaline group, Seignette's salt, and potassium-sodium niobate.

## Revendications

1. Dispositif d'électrodes implantable, notamment, pour des appareils cardiologiques, comme un stimulateur cardiaque, comprenant
- un corps d'électrode (3) étiré en longueur avec une extrémité proximale (4) et une extrémité distale (5),
- au moins une électrode (6, 7) pouvant être mise en contact avec le corps du support d'implant à l'extrémité distale (5) du corps d'électrode (3) ou à proximité, et
- au moins une ligne de contact électrique (8, 9) pour la mise en contact de l'électrode (6, 7),
- un dispositif de résonance électromécanique (10) raccordé à l'électrode (6, 7) ou à la ligne de contact (8, 9) pour la transformation de signaux de haute fréquence impulsés dans le dispositif d'électrodes sous forme d'oscillations acoustiques mécaniques (12)
**caractérisé en ce que**
- le dispositif de résonance électromécanique (10) est disposé du point de vue de la technique du circuit entre l'électrode (6) ou sa ligne de contact (8) et le corps (K) du support d'implant.

2. Dispositif d'électrodes selon la revendication 1, **caractérisé en ce que** le dispositif de résonance électromécanique (10) présente un élément de résonance piézo-cristallin (11).

3. Dispositif d'électrodes au moins selon la revendication 2, **caractérisé en ce que** l'élément de résonance piézo-cristallin (11) présente un dimensionnement géométrique (RD, SD) qui correspond à la demie longueur d'onde ou à un nombre impair de fois la demie longueur d'onde des oscillations acoustiques mécaniques dans un domaine de fréquences de 10 MHz à 400 MHz.

4. Dispositif d'électrodes au moins selon la revendication 2, **caractérisé en ce que** l'élément de résonance piézo-cristallin (11) est un élément piézo-cristallin en forme de manchon (13).

5. Dispositif d'électrodes selon la revendication 4, **caractérisé en ce que** l'épaisseur annulaire (RD) de l'élément piézo-cristallin en forme de manchon (13) correspond à la demie longueur d'onde de l'oscillation acoustique mécanique.

6. Dispositif d'électrodes au moins selon la revendication 1, **caractérisé en ce que** l'élément de résonance piézo-cristallin (11) est formé par un film piézo-cristallin (19, 22) en polyfluorure de vinylidène PVDF.

7. Dispositif d'électrodes selon la revendication 6, **caractérisé en ce que** le film piézo-cristallin (19, 22) est recouvert d'une couche de métal (20, 23).

8. Dispositif d'électrodes selon la revendication 7, **caractérisé en ce que** le film piézo-cristallin (19, 22), ensemble avec la couche de métal (20, 23), est conçu sous la forme d'une construction en sandwich avec au moins deux couches alternées de film et de métal (19, 20, 22, 23).

9. Dispositif d'électrodes selon la revendication 7 ou 8, **caractérisé en ce que** le film piézo-cristallin (19, 22), avec la couche de métal (20, 23), est enroulé plusieurs fois autour d'un support, notamment autour d'un manchon de tête (14) d'une électrode à pointe (6) pour la formation du dispositif de résonance électromécanique (10).

10. Dispositif d'électrodes selon la revendication7 ou 8, **caractérisé en ce que** la couche de métal (20) est munie d'une borne de connexion (21) pour la mise en contact.

11. Dispositif d'électrodes selon l'une des revendications précitées avec un manchon de tête (24) dans la région de l'électrode distale (6, 7), **caractérisé en ce que** le dispositif de résonance électromécanique (10) est rapporté à l'extérieur sur le manchon de tête (14) ou forme une section du manchon de tête (14).

12. Dispositif d'électrodes selon l'une des revendications précitées, **caractérisé en ce que** l'élément de résonance piézo-cristallin (11) est constitué totalement ou partiellement d'un matériau choisi dans le groupe : des SiO₂ anisotropes (quartz α, verre), du nitrure d'aluminium, du polyfluorure de vinylidène, du titanate de baryum, du niobate de lithium, de l'ortho-phosphate de gallium, de la berlinite, de minéraux du groupe de la tourmaline, du sel de Seignette ou de niobate de potassium et de sodium.
